# FASCICULE DE BREVET EUROPEEN

(11) **EP 0 754 173 B1**
(45) Date de publication et mention de la délivrance du brevet: **14.04.1999**
(21) Numéro de dépôt: 96900687.3
(22) Date de dépôt: 07.02.1996
(51) Int. Cl.: C07C 67/60, A61K 31/22

(54) **PROCEDE DE PURIFICATION DE LA DIACETYLRHEINE**
VERFAHREN ZUR REINIGUNG VON DIACETYLRHEIN
METHOD FOR PURIFYING DIACETYL RHEIN

(30) Priorité: 07.02.1995 CH 342/95
(43) Date de publication de la demande: 22.01.1997
(73) Titulaire: Steba Beheer B.V., 2514 JB La Haye (NL)
(72) Inventeur: COHEN, Avraham, 64587 Tel Aviv (IL)
(74) Mandataire: Hranitzky, Wilhelm Max
(86) Numéro de dépôt international: IB9600093
(87) Numéro de publication internationale: WO9624572

(56) Documents cités:
- EP-A- 0 243 968
- EP-A- 0 636 602
- DE-A- 2 711 493
- DE-A- 4 120 989
- DE-A- 4 120 990

## Description

La présente invention concerne un nouveau procédé de purification de la diacétylrhéine et, plus particulièrement, un procédé permettant d'obtenir de la diacétylrhéine ayant un degré de pureté suffisant pour être utilisable dans la préparation de médicaments.

L'arthrose est une maladie largement répandue à travers le monde, touchant plus particulièrement les personnes âgées, se manifestant par une dégradation des tissus cartilagineux notamment au niveau des articulations et des vertèbres, entraînant des effets douloureux et invalidants chez les personnes qui en souffrent. Le vieillissement de la population dans les pays occidentaux rend plus nécessaires encore les traitements destinés à soulager les personnes souffrant de cette maladie.

L'administration de médicaments anti-inflammatoires non stéroidiens et d'analgésiques, par exemple l'indométhacine, le naproxène et l'aspirine, permet généralement de soulager temporairement le patient, mais n'entraîne pas de réelle guérison. Plus récemment, on a montré que certains dérivés d'acide anthraquinone carboxylique et, plus particulièrement, la diacétylrhéine, procurent de bons résultats dans le traitement de certaines formes d'arthrite et de l'arthrose. Ainsi, on a constaté que la diacétylrhéine, administrée par voie orale, possède une activité anti-inflammatoire et analgésique efficace dans le traitement de l'arthrose, sans que l'on ait observé d'effet secondaire néfaste.

Des dérivés d'acide 1,8-dihydroxy-anthraquinone-3-carboxylique, incluant la diacétylrhéine, sont décrits dans le brevet FR-A-2.508.798.

Le brevet EP-A-243.968 décrit des sels alcalins de diacétylrhéine, tels que les sels de sodium et de potassium, obtenus par dissolution de la diacétylrhéine dans un mélange d'acétone et d'eau, addition de triéthylamine et salification par un acide organique faible en milieu alcoolique (isobutanol). Ces sels de sodium et de potassium sont utilisés pour la fabrication de solutions destinées à l'administration par voie intraveineuse, intramusculaire ou intrapéritonéale.

La diacétylrhéine est une substance d'origine végétale, obtenue à partir de produits d'extraction d'aloes ou de feuilles de séné. Ainsi par exemple, elle peut être préparée par acétylation de l'acide 1,8-dihydroxy-anthraquinone-3-carboxylique (rhéine), que l'on peut obtenir à partir de sennosides extraits de feuilles de séné. Cependant, la diacétylrhéine ainsi obtenue contient d'importantes quantités d'aloe-emodine, produit secondaire indésirable, provenant des matières de départ, et sa purification est difficile. On sait aussi préparer la diacétylrhéine par acétylation de barbaloïne, suivie d'une oxydation par l'oxyde de chrome, mais cette technique, elle aussi, procure un produit final présentant un taux élevé d'aloe-emodine, et elle entraîne la formation de résidus chromiques qui sont des déchets très difficiles à éliminer par des techniques usuelles.

Les brevets DE-A-4.120.989 et DE-A-4.120.990 décrivent des procédés de préparation de diacétylrhéine à partir de sennosides pouvant contenir de l'aloe-emodine, comportant plusieurs étapes consistant à oxyder des rhéine-9-anthrone-8-glucosides, à séparer le reste glucose et à acétyler, les procédés comportant en outre une étape de séparation liquide-liquide. Bien que la pureté de la diacétylrhéine obtenue soit améliorée, le taux d'aloeemodine dans le produit obtenu reste relativement important (près de 20 ppm), ce qui peut entraîner la nécessité d'effectuer des purifications complémentaires longues et coûteuses.

La présente invention a pour objet un procédé permettant de préparer de la diacétylrhéine d'excellente pureté, avec un bon rendement.

L'invention a également pour objet un procédé de purification de diacétylrhéine, à partir d'une composition contenant diverses impuretés et notamment des dérivés d'aloe-emodine, pour obtenir une diacétylrhéine exempte d'aloe-emodine.

La diacétylrhéine obtenue par le procédé suivant la présente invention est d'une pureté telle qu'elle est substantiellement exempte d'aloe-emodine, le taux effectif d'aloe-emodine dans le produit final n'étant pas décelable par les techniques connues.

Le produit de départ utilisé dans le procédé de l'invention est une diacétylrhéine contenant diverses impuretés et, en particulier, des quantités relativement importantes de dérivés d'aloe-emodine, par exemple plus de 50 ppm, et même dans certains cas les quantités de dérivés d'aloe-emodine peuvent être de l'ordre de 150 à 200 ppm. L'expression "dérivés d'aloe-emodine" signifie non seulement l'aloe-emodine elle-même mais des dérivés tels que la triacétyl-aloe-emodine.

Le procédé de purification de la diacétylrhéine conforme à la présente invention consiste à préparer un sel soluble de la diacétylrhéine, puis à effectuer une hydrolyse en milieu faiblement acide pour provoquer la précipitation. Il comprend essentiellement les étapes consistant à:
(a) mettre la diacétylrhéine en suspension dans un mélange solvant organique / eau;
(b) ajouter une amine tertiaire;
(c) ajouter à la solution obtenue un sel d'acide de métal alcalin ou alcalino-terreux;
(d) effectuer une hydrolyse en milieu faiblement acide;
(e) recueillir la diacétylrhéine purifiée, par filtration.

En utilisant le procédé conforme à la présente invention, on obtient une diacétylrhéine d'excellente pureté avec un rendement supérieur à 90 %, pouvant atteindre 93 à 95 % en choisissant les conditions opératoires de manière appropriée, comme indiqué ci-après. Ce rendement peut aisément être porté à 96 % environ en recyclant les produits de filtration.

D'autre part, la pureté de la diacétylrhéine ainsi obtenue est remarquable, car le taux d'aloe-emodine et de dérivés d'aloe-emodine provenant des matières premières n'est pas décelable, le seuil de détection des appareils d'analyse classiquement utilisés, tels que les appareillages de chromatographie HPLC, se situant au niveau du ppm. Ainsi la diacétylrhéine purifiée obtenue par le procédé de la présente invention est substantiellement exempte d'aloe-emodine. Ce résultat est particulièrement avantageux par rapport aux taux habituellement relevés dans les procédés connus de la technique, qui sont généralement largement supérieurs à 20 ppm.

La diacétylrhéine, dans l'étape (a), est mise en suspension dans un mélange solvant organique / eau dans un rapport en poids compris en 10/1 et 50/1, et, de préférence, entre 20/1 et 30/1. Conformément au procédé de l'invention, on utilise une quantité minimale d'eau. L'absence d'eau défavorise la purification, tandis que l'utilisation d'une quantité d'eau supérieure aux valeurs indiquées ci-dessus aurait pour conséquence une diminution du rendement.

Le solvant organique peut être choisi parmi l'acétone, la méthyl éthyl cétone, l'éthanol et le diméthylacétamide.

L'amine tertiaire utilisée dans l'étape (b) est de préférence la triéthylamine et, plus préférentiellement, de la triéthylamine en mélange avec de l'acétone, dans un rapport molaire inférieur à 1, de préférence compris entre 0,3 et 0,6. D'autre part, le rapport molaire de l'amine tertiaire à la diacétylrhéine est de préférence compris entre 1 et 1,2. Un excès plus important d'amine n'améliore ni le rendement, ni la pureté du produit obtenu. En opérant comme indiqué ci-dessus, conformément à la présente invention, on obtient une dissolution complète du sel d'amine, ce qui permet d'améliorer sensiblement le rendement industriel.

Le sel utilisé dans l'étape (c) est choisi parmi l'acétate, l'éthyl-hexanoate et le propionate de sodium ou de potassium. On utilise de préférence l'acétate de potassium, en excès, le rapport molaire acétate / diacétylrhéine étant de préférence supérieur à 1,2 et, plus préférentiellement, compris entre 1,5 et 2.

L'étape d'hydrolyse est effectuée de préférence à un pH compris entre 3 et 5, au moyen d'acide chlorhydrique ou d'acide sulfurique, en opérant à une température comprise entre 2 et 15 °C et, plus préférentiellement, entre 5 et 8 °C. Un pH plus élevé, supérieur à 5, doit être évité car il entraîne une dégradation en produits monoacétylés.

Le procédé conforme à la présente invention est tout particulièrement avantageux car il est utilisable pour préparer industriellement de la diacétylrhéine de qualité pharmaceutique.

La diacétylrhéine purifiée ainsi obtenue se présente sous forme de cristaux utilisables directement dans les procédés usuels de mise sous forme pharmaceutique, par exemple pour la fabrication de gélules pour administration par voie orale.

L'efficacité de la diacétylrhéine purifiée obtenue par le procédé de l'invention a été vérifiée sur les modèles expérimentaux usuels. Les résultats des essais toxicologiques (DL₅₀ par voie orale chez le rat et la souris) sont conformes à ceux décrits dans la littérature. Les essais cliniques ont confirmé l'efficacité des compositions pharmaceutiques, pour administration par voie orale, contenant la diacétylrhéine ainsi purifiée, plus particulièrement dans le traitement de l'arthrose.

Les exemples suivants illustrent l'invention sans en limiter la portée.

### Exemple 1

Dans un mélange d'acétone et d'eau déminéralisée (540 l d'acétone, 25 l d'eau) on met en suspension 50 kg de diacétylrhéine contenant des impuretés et, plus particulièrement, de l'aloe-emodine et de la triacétyl-aloe-emodine (taux supérieur à 150 ppm).

La suspension est maintenue sous agitation à température ambiante pendant 10 à 15 minutes, puis, après homogénéisation, elle est portée à une température de 40 - 45 °C.

On ajoute ensuite un mélange de triéthylamine et d'acétone (15 kg de triéthylamine pour 19 kg d'acétone) et on maintient sous agitation pendant environ 10 minutes jusqu'à complète dissolution du sel d'ammonium de diacétylrhéine formé. La solution est ensuite filtrée pour éliminer toutes traces d'impuretés solides.

On ajoute 25 kg d'acétate de potassium sous forme de poudre, on ramène la température du milieu réactionnel à 10 °C et on maintient sous agitation pendant environ 1 heure.

On recueille par filtration le sel de potassium de diacétylrhéine ainsi formé, que l'on lave par 150 l d'acétone. Puis le sel est dissous dans 2'000 l d'eau déminéralisée, et on ajoute de l'acide sulfurique à 33 % jusqu'à obtenir un pH égal à 5, puis on abaisse lentement le pH jusqu'à 4.

Après refroidissement à environ 10 °C, la diacétylrhéine formée par hydrolyse est recueillie par filtration, lavée à l'eau déminéralisée jusqu'à pH neutre, et le produit est ensuite séché en étuve à 60-80 °C environ de manière à obtenir un taux d'humidité inférieur à 1 %.

Le rendement en diacétylrhéine purifiée est de 93 %.

Les filtrats du sel de diacétylrhéine sont acidifiés, concentrés pour éliminer l'acétone, puis repris par 150 l d'eau déminéralisée et refroidis à 10 °C pour récupérer la diacétylrhéine brute qui est purifiée comme indiqué ci-dessus. On porte alors le rendement global de la purification à 96 %.

Le taux d'aloe-emodine dans le produit final purifié est vérifié par chromatographie (méthode d'extraction : l'aloe-emodine, ainsi que celle provenant de la transformation de la triacétyl-aloe-emodine en milieu basique, est extraite de la diacétylrhéine à analyser; l'extrait est ensuite analysé en HPLC sur colonne C 18 à 254 nm). Le résultat est inférieur au seuil de détection, qui est de l'ordre de ppm.

### Exemple 2

On procède comme dans l'exemple 1, en utilisant comme matière de départ une diacétylrhéine brute contenant environ 60 ppm d'aloe-emodine, mais en remplaçant l'acétate de potassium par le propionate de potassium, préparé à partir de l'acide correspondant en solution aqueuse.

Le rendement, avant recyclage des filtrats, est de 85 %.

Le taux d'aloe-emodine dans le produit purifié reste inférieur au seuil de détection de l'appareillage HPLC.

### Exemple 3

On procède comme dans l'exemple 1 ci-dessus, mais en utilisant, pour l'hydrolyse du sel d'ammonium, de l'acide chlorhydrique à la place de l'acide sulfurique, et en remplaçant le mélange de solvants acétone l eau déminéralisée, par un mélange éthanol / eau.

On obtient des résultats équivalents à ceux indiqués ci-dessus.

### Exemple 4

On procède comme indiqué dans l'exemple 1 ci-dessus, mais en utilisant une quantité d'eau doublée par rapport à celle de l'exemple 1 pour la préparation de la suspension de diacétylrhéine (50 l au lieu de 25 l).

On constate alors que le rendement est légèrement inférieur (80 % au premier cycle, que l'on peut améliorer au recyclage) mais la pureté n'est pas modifiée (taux inférieur au seuil de détection).

## Revendications

1. Procédé de purification de la diacétylrhéine, caractérisé en ce qu'il comprend les étapes consistant à :
(a) mettre la diacétylrhéine en suspension dans un mélange solvant organique / eau;
(b) ajouter une amine tertiaire;
(c) ajouter à la solution obtenue un sel d'acide de métal alcalin ou alcalino-terreux;
(d) effectuer une hydrolyse en milieu faiblement acide;
(e) recueillir la diacétylrhéine purifiée, par filtration.

2. Procédé selon la revendication 1, caractérisé en ce que l'hydrolyse est effectuée à un pH compris entre 3 et 5.

3. Procédé selon l'une quelconque des revendications 1 et 2, caractérisé en ce que l'hydrolyse est effectuée au moyen d'acide chlorhydrique ou d'acide sulfurique.

4. Procédé selon l'une quelconque des revendications 1 à 3, caractérisé en ce que l'hydrolyse est effectuée à une température comprise entre 2 et 15 °C.

5. Procédé selon la revendication 4, caractérisé en ce que la température est comprise entre 5 et 8 °C.

6. Procédé selon la revendication 1, caractérisé en ce que la diacétylrhéine, dans l'étape (a), est mise en suspension dans un mélange solvant organique / eau dans un rapport en poids compris entre 10/1 et 50/1.

7. Procédé selon la revendication 6, caractérisé en ce que le solvant organique est choisi parmi l'acétone, la méthyl éthyl cétone, l'éthanol et le diméthylacétamide.

8. Procédé selon la revendication 1, caractérisé en ce que l'amine tertiaire utilisée dans l'étape (b) est la triéthylamine.

9. Procédé selon la revendication 8, caractérisé en ce que la triéthylamine est utilisée en mélange avec de l'acétone dans un rapport molaire triéthylamine / acétone compris entre 0,3 et 0,6.

10. Procédé selon la revendication 1, caractérisé en ce que le sel utilisé dans l'étape (c) est choisi parmi l'acétate, l'éthyl-hexanoate et le propionate de sodium ou de potassium.

11. Procédé selon l'une quelconque des revendications 1 à 4, caractérisé en ce que l'hydrolyse de l'étape (d) est réalisée en milieu acide sulfurique ou chlorhydrique dans un mélange de solvants acétone / eau ou éthanol /eau.

## Patentansprüche

1. Verfahren zur Erhöhung der Reinheit von Diacetylrhein, dadurch gekennzeichnet, dass es die Verfahrensschritte enthält, die darin bestehen:
(a) das Diacetylrhein in einer Mischung organisches Lösungsmittel/Wasser in Suspension zu bringen;
(b) ein tertiäres Amin hinzuzufügen;
(c) der erhaltenen Lösung ein Salz einer Säure eines Alkalimetalls oder eines Erdalkalimetalls hinzuzufügen;
(d) eine Hydrolyse in schwach saurem Medium durchzuführen;
(e) das gereinigte Diacetylrhein durch Filtrierung zu gewinnen.

2. Verfahren nach Patentanspruch 1, dadurch gekennzeichnet, dass die Hydrolyse bei einem pH Wert, der zwischen 3 und 5 liegt, durchgeführt wird.

3. Verfahren nach einem der Patentansprüche 1 und 2, dadurch gekennzeichnet, dass die Hydrolyse mit Hilfe von Salzsäure oder Schwefelsäure durchgeführt wird.

4. Verfahren nach einem der Patentansprüche 1 bis 3, dadurch gekennzeichnet, dass die Hydrolyse bei einer Temperatur zwischen 2° und 15° C durchgeführt wird.

5. Verfahren nach Patentanspruch 4, dadurch gekennzeichnet, dass die Temperatur zwischen 5° und 8° C liegt.

6. Verfahren nach Patentanspruch 1, dadurch gekennzeichnet, dass das Diacetylrhein im Verfahrensschritt (a) in einer Mischung organisches Lösungsmittel/Wasser mit einem Gewichtsverhältnis zwischen 10/1 und 50/1 in Suspension gebracht wird.

7. Verfahren nach Patentanspruch 6, dadurch gekennzeichnet, dass das organische Lösungsmittel unter Aceton, Methylethylketon, Ethanol und Dimethylacetamid gewählt wird.

8. Verfahren nach Patentanspruch 1, dadurch gekennzeichnet, dass das im Verfahrensschritt (b) verwendete tertiäre Amin Triethylamin ist.

9. Verfahren nach Patentanspruch 8, dadurch gekennzeichnet, dass das Triethylamin in Mischung mit Aceton in einem Molverhältnis Triethylamin/Aceton, das zwischen 0,3 und 0,6 liegt, verwendet wird.

10. Verfahren nach Patentanspruch 1, dadurch gekennzeichnet, dass das in Verfahrensschritt (c) verwendete Salz unter Natrium- oder Kali-Acetat, Ethylhexanoat und Propionat gewählt wird.

11. Verfahren nach einem der Patentansprüche 1 bis 4, dadurch gekennzeichnet, dass die Hydrolyse gemäss Verfahrensschritt (d) in einem Schwefelsäure- oder Salzsäuremedium mit einer Lösungsmittelmischung Aceton/ Wasser oder Ethanol/Wasser durchgeführt wird.

## Claims

1. A method for purifying diacetylrhein, characterized in that it includes the steps of :
(a) suspending the diacetylrhein in a mixture of organic solvent / water;
(b) adding a tertiary amine;
(c) adding to the solution obtained, a salt of an acid and of an alkali metal or of an alkaline-earth metal;
(d) carrying out a hydrolysis in a weakly acidic medium;
(e) collecting the purified diacetylrhein, by filtration.

2. A method according to claim 1, characterized in that the hydrolysis is carried out at a pH comprised between 3 and 5.

3. A method according to any one of claims l and 2, characterized in that the hydrolysis is carried out by means of hydrochloric acid or sulphuric acid.

4. A method according to any one of claims l to 3, characterized in that the hydrolysis is carried out at a temperature comprised between 2 and 15°C.

5. A method according to claim 4, characterized in that the temperature is comprised between 5 and 8°C.

6. A method according to claim 1, characterized in that the diacetylrhein in step (a) is suspended in a mixture of organic solvent /water in a weight ratio comprised between 10/1 and 50/1.

7. A method according to claim 6, characterized in that the organic solvent is selected from acetone, methylethylketone, ethanol and dimethylacetamide.

8. A method according to claim 1, characterized in that the tertiary amine used in step (b) is triethylamine.

9. A method according to claim 8, characterized in that the triethylamine is used in a mixture with acetone in a molar ratio triethylamine / acetone comprised between 0.3 and 0.6.

10. A method according to claim 1, characterized in that the salt used in step (c) is selected from sodium or potassium acetate, ethylhexanoate and propionate.

11. A method according to any one of claims l to 4, characterized in that the hydrolysis of step (d) is carried out in a sulphuric acid or a hydrochloric acid medium with a solvent mixture acetone / water or ethanol /water.
